# EUROPÄISCHE PATENTSCHRIFT

(11) **EP 4 536 110 B1**
(45) Veröffentlichungstag und Bekanntmachung des Hinweises auf die Patenterteilung: **25.03.2026**
(21) Anmeldenummer: 23731995.9
(22) Anmeldetag: 05.06.2023
(51) Int. Cl.: A61B 17/221, A61F 2/91, A61F 2/966

(54) **MEDIZINISCHE VORRICHTUNG UND HERSTELLUNGSVERFAHREN**
MEDICAL DEVICE AND PRODUCTION METHOD
DISPOSITIF MÉDICAL ET PROCÉDÉ DE PRODUCTION

(30) Priorität: 08.06.2022 DE 102022114379
(43) Veröffentlichungstag der Anmeldung: 16.04.2025
(73) Patentinhaber: Acandis GmbH, 75177 Pforzheim (DE)
(72) Erfinder: LANGE, Alexander, 76135 Karlsruhe (DE)
(74) Vertreter: Meissner Bolte Partnerschaft mbB
(86) Internationale Anmeldenummer: PCT/EP2023/064940
(87) Internationale Veröffentlichungsnummer: WO 2023/237472

(56) Entgegenhaltungen:
- EP-A1- 1 000 590
- EP-A1- 3 827 762
- WO-A1-2013/178297
- WO-A2-02/22028
- CN-A- 108 042 176
- DE-A1- 102015 117 666
- US-A1- 2008 167 679

## Beschreibung

Die Erfindung betrifft eine medizinische Vorrichtung und ein Verfahren zur Herstellung einer medizinischen Vorrichtung. Eine medizinische Vorrichtung gemäß dem Oberbegriff des Patentanspruchs 1 ist beispielsweise aus der DE 10 2020 109 158 A1 bekannt.

Medizinische Vorrichtungen in Form von Thrombektomie-Devices, auch als Stent-Retriever bezeichnet, dienen zur schnellen und sicheren Rekanalisation von Blutgefäßen. Solche Thrombektomie-Devices weisen herkömmlicherweise ein hybrides Zelldesign mit einer rohrförmigen Gitterstruktur auf, bei der Stege einzelne Zellen begrenzen. Dadurch werden eine hervorragende Wandapposition und Thrombus-Integration erreicht. Beispielsweise ist aus der eingangs genannten DE 10 2020 109 158 A1 ein Thrombektomie-Device bekannt, welches die vorgenannten Eigenschaften aufweist. Das Thrombektomie-Device umfasst an einem proximalen Ende der Gitterstruktur einen Formschlussabschnitt, der zur Verbindung mit einem Transportdraht dient. Auf der gegenüberliegenden, distalen Seite weist die Gitterstruktur freie Stegenden, sogenannte Pins, auf, die mittels Crimphülsen miteinander fest verbunden sind.

Aus dem Stand der Technik ist bekannt, dass Gitterstrukturen von Thrombektomie-Devices häufig aus einem Formgedächtnismaterial, wie beispielsweise Nitinol, lasergeschnitten werden. Dabei kommen üblicherweise Laserschneidanlagen zum Einsatz, bei denen der Laserschnitt durch die Längsachse des rohrförmigen Formgedächtnismaterials verläuft. Daraus resultiert eine trapezförmige Querschnittform der einzelnen Pins. Dies hat den Nachteil, dass sich deren Position bei der Herstellung der Crimpverbindung willkürlich verändert. Die Wahrscheinlichkeit, dass sich die Crimpverbindung bei leichter mechanischer Belastung wieder lockert ist hier sehr hoch. Dies führt dazu, dass sich die Gitterstruktur verzieht und somit deutlich schwerer in einem Katheter zu bewegen ist bzw. sich leichter an Engstellen verhakt. Im schlimmsten Fall könnte sich die Crimpverbindung komplett lösen, sodass die Crimphülse im Blutgefäß verloren geht. Ein Thrombus könnte dadurch nicht mehr ordnungsgemäß aufgenommen und somit entfernt werden.

Ein weiterer Nachteil besteht darin, dass durch die unerwünscht veränderte axiale Lage der Pins beim Ladevorgang eines Katheters die Gitterstruktur des Thrombektomie-Devices aufbuckeln kann. Dies führt dazu, dass das Device nicht vollständig komprimierbar ist und beim Beladen oder Wiedereinziehen in den Katheter nach dem Einfangen des Thrombus ein erhöhter Reibungswiderstand zwischen dem eingeführten Device und der Innenwand des Katheters entsteht. Es ist im Extremfall auch möglich, dass beim Beladen oder beim Wiedereinziehen in den Katheter nach dem Einfangen des Thrombus das Thrombektomie-Device in dem Katheter feststeckt bzw. sich verhakt.

Dokumente EP 3 827 762 A1, WO 02/22028 A2, WO 2013/178297 A1, DE 10 2015 117666 A1, US 2008/167679 A1, EP 1 000 590 A1 und CN 108 042 176 A offenbaren weitere medizinische Vorrichtungen mit verschiedenen Gitterstrukturen.

Der Erfindung liegt daher die Aufgabe zu Grunde, eine medizinische Vorrichtung anzugeben, die durch einen verbesserten konstruktiven Aufbau eine erhöhte Funktionssicherheit aufweist und einen sicheren Be- und Entladevorgang eines Katheters ermöglicht. Der Erfindung liegt ferner die Aufgabe zu Grunde ein Verfahren zur Herstellung einer medizinischen Vorrichtung anzugeben.

Erfindungsgemäß wird diese Aufgabe im Hinblick auf die medizinische Vorrichtung durch den Gegenstand des Anspruchs 1 gelöst. Hinsichtlich des Verfahrens zur Herstellung einer medizinischen Vorrichtung wird die vorstehend genannte Aufgabe durch den Gegenstand des Anspruchs 20 gelöst.

Konkret wird die Aufgabe durch eine medizinische Vorrichtung zur intravaskulären Behandlung mit einer zumindest abschnittsweise rohrförmigen Gitterstruktur gelöst, die von einem radial komprimierten Zustand in einen radial expandierten Zustand überführbar ist und eine Vielzahl von zellenbildenden Stegen aufweist. Die Gitterstruktur umfasst an einem distalen Endabschnitt wenigstens zwei, insbesondere drei oder mehr, mit den Stegen verbundene Pins, die ein freies Ende aufweisen. Die Pins sind in einem ersten Längsabschnitt zur Fixierung der Pinquerschnittslage zumindest abschnittsweise flächig aneinander angepresst.

Zusätzlich oder alternativ sind die Pins in einem zweiten Längsabschnitt zur Fixierung der Pinlängslage miteinander formschlüssig verbunden.

Besonders bevorzugt kommt die medizinische Vorrichtung als Thrombektomie-Device, insbesondere Stent-Retriever, zum Einsatz.

Die Erfindung hat verschiedene Vorteile. Durch das flächige Anpressen der Pins in dem ersten Längsabschnitt ist die Lage der Pins in radialer Richtung, das heißt quer zu einer Längserstreckung der Pins, fixiert. Die Pins liegen flächig aneinander an, sodass diese in ihrer Querschnittslage exakt ausrichtbar und fixierbar sind. Dies hat den Vorteil, dass bei einem Aneinanderpressen der Pins, bspw. durch Crimpen, Kleben, Schweißen oder dergleichen, ein Verkippen der Pins und somit ein Verzerren bzw. Verdrehen der rohrförmigen Gitterstruktur verhindert ist.

Ein weiterer Vorteil der Erfindung besteht darin, dass die Pins zusätzlich oder alternativ in ihrer Pinlängslage fixiert sind. Dies erfolgt durch die formschlüssige Verbindung der Pins miteinander. Mit anderen Worten sind die Pins derart miteinander formschlüssig verbunden, dass die Längslage der Pins zueinander fest ist. Dies hat den Vorteil, dass die Lage der Pins in Pinlängsrichtung gesichert ist. Eine unerwünschte Relativbewegung der Pins in Pinlängsrichtung ist dadurch verhindert. Dies führt dazu, dass beim Einführen der medizinischen Vorrichtung in einen Katheter ein Aufbuckeln bzw. Aufwölben der Gitterstruktur verhindert wird. Die Gitterstruktur ist durch die Fixierung der Pinlängslage vollständig komprimierbar, sodass ein Einführen der medizinischen Vorrichtung in den Katheter erleichtert wird. Im Gebrauch kann die medizinische Vorrichtung verzugsfrei entfaltet werden, sodass zusätzlich eine gleichmäßige Performance erreicht wird. Die Erfindung ermöglicht somit eine genaue radiale und axiale Ausrichtung und Fixierung der Pins im distalen Endabschnitt, sodass eine erhöhte Funktionssicherheit und eine verbesserte Handhabung bereitgestellt sind.

Unter der Pinquerschnittslage ist die Lage der Pins quer zur Pinlängsrichtung, das heißt in radialer Richtung, zu verstehen. Die Pinquerschnittslage ist bevorzugt die Lage der Pins zueinander quer zur Pinlängsrichtung. Unter der Pinlängslage ist die Lage der Pins in Pinlängsrichtung, das heißt in axialer Richtung, zu verstehen. Die Pinlängslage ist bevorzugt die Lage der Pins zueinander in Pinlängsrichtung, die insbesondere zu einer Pinlängserstreckung parallel ist.

Zur formschlüssigen Verbindung greifen die Pins bevorzugt in dem zweiten Längsabschnitt ineinander ein. Die Pins greifen vorzugsweise quer zu ihrer Längsrichtung ineinander ein. Zusätzlich oder alternativ können die Pins in Längsrichtung ineinander eingreifen oder, ganz allgemein, formschlüssig miteinander verbunden sein.

Der erste Längsabschnitt und der zweite Längsabschnitt definieren zwei Bereiche der Pins entlang deren Längserstreckung. Die beiden Längsabschnitte grenzen bevorzugt in Pinlängsrichtung aneinander an. Es ist möglich, dass die beiden Längsabschnitte der Pins aneinandergereiht die Gesamtlänge der Pins definieren. Mit anderen Worten kann die Gesamtlänge der Pins durch die beiden Längsabschnitte gebildet sein. Bevorzugt sind die Pins, insbesondere in dem ersten und zweiten Längsabschnitt, zum freien Ende hin geradlinig ausgebildet. Die Pins verlaufen bevorzugt, insbesondere in dem ersten und zweiten Längsabschnitt, parallel.

Vorzugsweise liegen die Pins zumindest teilweise in dem ersten Längsabschnitts aneinander an. Die Pins können über die gesamte Länge des ersten Längsabschnitts aneinander anliegen. Alternativ können die Pins partiell, insbesondere über wenigstens eine Teillänge, in dem ersten Längsabschnitt aneinander anliegen.

Bevorzugt sind alle Pins in ihrer Form gleich ausgestaltet. Vorzugsweise weisen die Pins jeweils identische erste Längsabschnitte und/oder identische zweite Längsabschnitte auf.

Die freien Enden der Pins bilden vorzugsweise ein freies, distales Ende des distalen Endabschnitts der Gitterstruktur. Bevorzugt bilden die Pins in dem distalen Endabschnitt ein Bündel. Dabei kann das Bündel ein freies, distales Ende der Gitterstruktur bilden. In einer bevorzugten Ausführungsform weist die Gitterstruktur drei Pins auf, die in dem ersten Längsabschnitt flächig aneinandergepresst und/oder in dem zweiten Längsabschnitt miteinander formschlüssig verbunden sind. Alternativ kann die Gitterstruktur vier, fünf oder mehr Pins umfassen.

Vorzugsweise bilden die Pins ein geschlossenes Ende der Gitterstruktur. Konkreter bilden die Pins vorzugsweise als Bündel ein geschlossenes, distales Ende der Gitterstruktur. Die Pins schließen vorzugsweise an einen konisch zusammenlaufenden Bereich der rohrförmigen Gitterstruktur an und schließen die Gitterstruktur ab. Das geschlossene distale Ende der Gitterstruktur ermöglicht im Gebrauch ein besseres Einfangen von Thrombusfragmenten und verhindert ein Wiederhinausgleiten eines bereits im Inneren der Gitterstruktur aufgenommenen Thrombus.

Es wird darauf hingewiesen, dass im Rahmen der Patentanmeldung der Begriff "distal" eine vom Benutzer der medizinischen Vorrichtung abgewandte Seite bzw. entfernt liegende Seite der Gitterstruktur bedeutet. Der Begriff "proximal" bedeutet eine zum Benutzer hinweisende Seite der medizinischen Vorrichtung, d.h. eine näher am Benutzer liegende Seite.

Die rohrförmige Gitterstruktur der medizinischen Vorrichtung ist radial expandierbar bzw. komprimierbar. Dabei kann die rohrförmige Gitterstruktur selbstständig, beispielsweise unter Ausnutzung des Shape-Memory-Effekts, von einem radial komprimierten in einen radial expandierten Zustand überführt werden. Insofern ist die rohrförmige Gitterstruktur der medizinischen Vorrichtung vorzugsweise selbstexpandierend. Die Gitterstruktur kann abschnittsweise oder vollständig rohrförmig ausgebildet sein.

Bevorzugte Ausführungsformen der Erfindung sind in den Unteransprüchen angegeben.

Bei einer bevorzugten Ausführungsform weisen die Pins jeweils wenigstens eine radial innenliegende Anpressfläche auf, mit der die Pins aneinander anliegen, wobei die Anpressflächen so ausgerichtet sind, dass bei Einleitung einer Anpresskraft die Pins in ihrer Querschnittslage fixiert sind. Mit anderen Worten weisen die Pins jeweils wenigstens eine Anpressfläche auf, die auf einer Innenseite der Pins ausgebildet ist. Die Innenseite der Pins, insbesondere die radial innenliegende Pinseite, ist die jeweils einander zugewandte Seite der Pins quer zur Pinlängsrichtung, insbesondere in Querschnittsrichtung. Die Anpressfläche verläuft an den Pins in Pinlängsrichtung. Die Pins stehen durch die Anpressflächen miteinander in Kontakt. Die Pins liegen vorzugsweise durch die Anpressflächen direkt aneinander an. Zusätzlich ist die jeweilige Anpressfläche an den Pins so ausgebildet, dass die Querschnittslage der Pins zueinander bei Aufbringen einer Anpresskraft fixiert ist. Durch die das innenseitig flächige Anliegen ist eine exakte Ausrichtung der Pins zueinander sowie eine anschließende positionsgenaue Fixierung der Pins möglich.

Bei einer weiteren bevorzugten Ausführungsform weisen die Pins jeweils auf einer radial innenliegenden Pinseite zwei aufeinander zulaufende Anpressflächen zur Anlage zumindest eines benachbarten Pins auf. Die beiden Anpressflächen verlaufen in Längsrichtung an den Pins. Mit anderen Worten sind die beiden Anpressflächen derart ausgebildet, dass die Pins an deren Innenseite quer zur Pinlängsrichtung verjüngt sind. Oder anders gesagt, bilden die Anpressflächen auf der innenliegenden Pinseite Anpressschrägen, an denen jeweils ein benachbarter Pin mit einer dessen Anpressflächen, insbesondere Anpressschrägen, anliegt. Die Anordnung von zwei innenseitigen Anpressflächen hat den Vorteil, dass eine Positionsgenauigkeit bei der Ausrichtung sowie Fixierung der Pins erhöht ist.

Die Anpressflächen sind vorzugsweise geradflächig ausgebildet. Bevorzugt sind die aneinander anliegenden Anpressflächen von zwei benachbarten Pins komplementär ausgebildet.

Vorzugsweise weisen die Pins wenigstens eine radial außenliegende Krafteinleitfläche auf, wobei die radial innenliegende Anpressfläche in Bezug auf die Krafteinleitfläche schräg verläuft. Mit anderen Worten weisen die Pins jeweils wenigstens eine Fläche zur Einleitung einer Anpresskraft auf, die auf einer Außenseite der Pins ausgebildet ist. Die Außenseite der Pins ist die jeweils voneinander abgewandte Seite der Pins quer zur Pinlängsrichtung, insbesondere in Querschnittsrichtung. Über die Krafteinleitfläche wird im fixierten Zustand der Pins eine Anpresskraft von außen in die Pins eingeleitet, sodass ein Aneinanderpressen von zumindest zwei benachbarten Pins erfolgt. Durch die schrägverlaufende Anpressfläche wird eine Teilkomponente der eingeleiteten Anpresskraft in Richtung der Anpressfläche umgelenkt, sodass zumindest eine weitere Teilkomponente der Anpresskraft bevorzugt für eine weitere Anpressfläche zur Verfügung steht.

Die Krafteinleitfläche verläuft an den Pins in Pinlängsrichtung. Dies kann zumindest abschnittsweise erfolgen. Alternativ kann die Krafteinleitfläche über die gesamte Länge der Pins, insbesondere des ersten Längsabschnitts verlaufen. Die Krafteinleitfläche weist vorzugsweise eine Krümmung auf. Mit anderen Worten kann die Krafteinleitfläche gewölbt ausgebildet sein. Die Wölbung bzw. Krümmung ist vorzugsweise konvex. Es ist möglich, dass alternativ oder zusätzlich die Krafteinleitfläche zumindest teilweise geradflächig, insbesondere zumindest teilweise frei von einer Krümmung sein kann.

Besonders bevorzugt liegt bei dieser Ausführungsform die Krafteinleitfläche den zwei radial innenliegenden Anpressflächen gegenüber, wobei die beiden Anpressflächen in Bezug auf die Krafteinleitfläche schräg verlaufen. Mit anderen Worten sind die radial innenliegenden Anpressflächen in Bezug auf die Krafteinleitfläche winkelig ausgerichtet. Bevorzugt weisen die beiden Anpressflächen denselben Winkel in Bezug auf die Krafteinleitfläche auf, wobei diese in einer entgegengesetzten Richtung auf der Innenseite der Pins verlaufen. Dies hat den Vorteil, dass bei Einleitung einer Anpresskraft in den jeweiligen Pin die Anpresskraft in zwei gleich große Kraftkomponenten unterteilt wird und diese jeweils in Richtung einer der beiden Anpressflächen umgelenkt wird. Es erfolgt somit eine gleichmäßige Kraftverteilung an die beiden Anpressflächen, sodass eine stabile Fixierung der Pins erfolgt.

Bei einer bevorzugten Ausführungsform weisen die Pins auf deren Innenseite eine Schmalfläche auf. Mit anderen Worten umfassen die Pins jeweils eine radial innenliegende Spitze auf, die abgeflacht ist. Vorzugsweise laufen die beiden radial innenliegenden Anpressflächen auf die Schmalfläche zu. Die beiden Anpressflächen schließen bevorzugt an die Schmalfläche an. Die Schmalfläche verläuft vorzugsweise in Pinlängsrichtung, insbesondere zumindest im ersten Pinlängsabschnitt. Die Schmalfläche ist vorzugsweise ein Abschnitt einer Innenfläche (eines Innendurchmessers) eines geschnittenen, insbesondere lasergeschnittenen, rohrförmigen Ausgangsmaterials. Diese Ausführungsform hat den Vorteil, dass die Pins in ihrer Querschnittslage aufgrund verbesserter Passungseigenschaften sicherer positionierbar sind.

Es ist möglich, dass die Pins auf deren Innenseite jeweils eine Längskante aufweisen, auf die die beiden Anpressflächen zulaufen. Anders gesagt, weisen die Pins jeweils eine radial innenliegende Längskante auf, die frei von einer Abflachung ist. Hier grenzen die beiden Anpressflächen an der Längskante aneinander an.

Bei einer bevorzugten Ausführungsform umfassen die Pins, insbesondere das Bündel, ein gemeinsames Zentrum, das radial innenliegt, wobei zumindest eine Flächennormale FN der Krafteinleitfläche des jeweiligen Pins durch das gemeinsame Zentrum verläuft. Das gemeinsame Zentrum kann einen Mittelbereich bilden, der zwischen den Pininnenseiten vorgesehen ist. Alternativ kann das gemeinsame Zentrum ein Mittelpunkt sein, der auf einer gemeinsamen Mittellängsachse der Pins, insbesondere des Bündels liegt.

Im Rahmen der Anmeldung ist die Flächennormale FN eine gedachte Gerade, die an einem bestimmten Punkt normal auf der Krafteinleitfläche steht. Die Flächennormale FN verläuft quer zur Pinlängsrichtung. Die Flächennormale FN entspricht der Wirklinie der eingeleiteten Anpresskraft im Bereich der Krafteinleitfläche. Die Flächennormalen FN der Krafteinleitflächen aller Pins schneiden sich bevorzugt im gemeinsamen Zentrum. Besonders bevorzugt bildet der gemeinsame Mittelpunkt einen Schnittpunkt, an dem sich die Flächennormalen FN einer jeden Krafteinleitfläche schneiden. Bei dieser Ausführungsform ist vorteilhaft, dass eine homogene Kraftverteilung zwischen den Pins erfolgt, sodass ein Verrutschen der Pins in radialer Richtung verhindert wird. Mit anderen Worten wird durch die gleichmäßige Kraftverteilung die jeweilige vorbestimmte Querschnittslage der Pins sichergestellt, d.h. fixiert.

Bevorzugt weisen die Pins wenigstens eine gemeinsame Außenkontur auf, die im Querschnitt annähernd einem regelmäßigen Polygon entspricht. Das regelmäßige Polygon kann ein gleichseitiges Dreieck, ein gleichseitiges Viereck oder ein gleichseitiges Vieleck, wie beispielsweise ein Sternpolygon sein. Das regelmäßige Polygon kann imaginär sein, d.h. die gemeinsame Außenkontur kann unterbrochen sein, sodass zumindest ein Teilabschnitt des regelmäßigen Polygons imaginär ist. Es ist allgemein bekannt, dass die Ecken eines regelmäßigen Polygons auf einem gemeinsamen Kreis liegen und dadurch einen gleichen Mittelpunktswinkel aufweisen. Konkret bedeutet dies, dass bei dieser Ausführungsform die Flächennormalen FN durch einen gemeinsamen Mittelpunkt verlaufen, sodass eine homogene Kraftverteilung möglich ist.

Die Pins sind bevorzugt miteinander sternförmig angeordnet. Mit anderen Worten weisen die Pins als Bündel bevorzugt eine Sternformanordnung auf. Dies ermöglicht neben einer verbesserten Kraftverteilung innerhalb des Bündels, eine vereinfachte Fixierung der Pins miteinander, bspw. durch eine Crimphülse, eine Klebehülse und/oder Schweißnähte. Durch die Sternanordnung kann somit eine stabile und robuste Verbindung der Pins realisiert werden.

Bevorzugt umfassen die Pins im Querschnitt wenigstens ein Ringsegment. Besonders bevorzugt bilden die Pins im Querschnitt jeweils ein Ringsegment. Die Pins füllen bevorzugt im Querschnitt des Bündels einen in Umfangsrichtung geschlossenen Kreisring aus. Sind drei Pins vorgesehen, weisen die Pins bevorzugt jeweils ein Ringsegment im Wesentlichen von 120 Grad auf. Sind mehr als drei Pins vorgesehen, weisen die Pins bevorzugt jeweils ein Ringsegment mit derselben Winkelerstreckung auf. Andere, insbesondere ungleichmäßige Ringsegmentaufteilungen sind möglich. Ferner können die Pins alternativ zur Ringsegmentvariante im Querschnitt kreissegmentförmig ausgebildet sein.

Bei einer bevorzugten Ausführungsform weist die medizinische Vorrichtung wenigstens ein Verbindungselement auf, das an den Pins außen angeordnet ist und die Pins, insbesondere in dem ersten Längsabschnitt, radial aneinander anpresst. Besonders bevorzugt ist das Verbindungselement eine Hülse, insbesondere Crimphülse, die mit den Pins zu deren Fixierung vercrimpt ist. Bei dieser Ausführungsform ist die Hülse bevorzugt mit den Pins derart vercrimpt, dass die Hülse mit einer Innenseite an den Krafteinleitflächen der Pins anliegt. Dadurch ergibt sich eine homogene Krafteinleitung einer radialen Haltekraft der Hülse in die Pins.

Die Hülse ist in einem Bereich der Krafteinleitflächen derart deformiert, dass diese jeweils eine Anpresskraft über die Krafteinleitflächen in die Pins einleitet. Die Innenseite der Hülse kann mit den Krafteinleitflächen jeweils in Punktkontakt oder Linienkontakt stehen. Bevorzugt steht die Innenseite der Hülse mit den Krafteinleitflächen jeweils in flächigem Kontakt. Dadurch werden die Pins lagefest fixiert.

Alternativ oder zusätzlich kann das Verbindungselement eine Hülse sein, die mit den Pins verklebt ist. Es ist denkbar, dass die Pins alternativ oder zusätzlich durch wenigstens eine Schweißnaht, insbesondere in dem ersten Längsabschnitt, miteinander stoffschlüssig verbunden sind. Bevorzugt ist dabei wenigstens eine Schweißnaht je zwei benachbarte Pins vorgesehen. Mit anderen Worten sind je zwei benachbarte Pins durch eine Schweißnaht miteinander fest verbunden. Die Schweißnaht kann alternativ ein Schweißpunkt sein.

Vorzugsweise weist das Verbindungselement einen Außenumfang auf, der im Wesentlichen kreisförmig, insbesondere rund ist. Mit anderen Worten weist das Verbindungselement bevorzugt eine im Wesentlichen kreisförmige, insbesondere runde, Hüllkurve auf. Dies hat den Vorteil, dass die Schiebbarkeit der medizinischen Vorrichtung bspw. Einführen in einen Katheter oder Herausführen aus dem Katheter verbessert ist.

Das Verbindungselement kann umlaufend geschlossen oder teilweise offen sein. Konkret kann die Hülse umlaufend geschlossen oder teilweise offen sein. Die geschlossene Hülse hat den Vorteil, dass beim Gebrauch die Wahrscheinlichkeit des Verlusts der Hülse in dem Blutgefäß verringert ist. Demgegenüber hat die teilweise offene Hülse den Vorteil, dass eine Notöffnung der Hülse bei Überlastung durchgeführt werden kann. Insbesondere ist es von Vorteil, wenn die offene Hülse an zumindest einem der Pins angeschweißt ist, sodass die Hülse im Falle der Notöffnung, aber auch bei einem unerwünschten Lösen der offenen Hülse von den Pins in dem Gefäß nicht verloren geht. Alternativ oder zusätzlich kann die Hülse mit wenigstens einem der Pins verklebt sein.

Bei einer bevorzugten Ausführungsform weisen die Pins, insbesondere in dem zweiten Längsabschnitt, jeweils wenigstens einen ersten Fortsatz und wenigstens eine gegenüber angeordnete Ausnehmung auf, wobei der erste Fortsatz des einen Pins in die Ausnehmung des benachbarten Pins zumindest abschnittsweise eingreift. Durch das Eingreifen des ersten Fortsatzes des einen Pins in die Ausnehmung des anderen Pins erfolgt eine exakte Längsausrichtung der Pins miteinander. Die Pins sind somit vor dem aneinander Anpressen im ersten Längsabschnitt in ihrer Längslage genau positionierbar sowie im aneinander angepressten Zustand in ihrer Längslage fixiert. Dadurch sind die Pins in Längsrichtung miteinander positionsfest verbunden. Es kann somit keine Relativbewegung der Pins erfolgen, wodurch ein Verziehen der Gitterstruktur und folglich ein Aufbuckeln bspw. beim Einführen in einen Katheter verhindert wird.

Der erste Fortsatz ist bevorzugt ein integraler Bestandteil des jeweiligen Pins. Der erste Fortsatz erstreckt sich vorzugsweise in radialer Richtung, d.h. quer zur Pinlängsrichtung. Es ist möglich, dass die Pins jeweils mehr als einen ersten Fortsatz und/oder mehr als eine Ausnehmung aufweisen. Bevorzugt sind der erste Fortsatz und die Ausnehmung in radialer Richtung an dem jeweiligen Pin gegenüber angeordnet. Der erste Fortsatz und die Ausnehmung sind vorzugsweise durch Laserschneiden gebildet. Besonders bevorzugt kommt dazu eine 4-Achs-Laserschneidanlage zum Einsatz, die es ermöglicht, einen exzentrischen Laserschnitt durchzuführen. Konkret kann die Laserschneidanlage eine 4-Achs-Rohrlaserschneidanlage sein.

Die Ausnehmung ist vorzugsweise in Längsrichtung des Pins von wenigstens zwei zweiten Fortsätzen begrenzt, die jeweils einen Längsanschlag für den ersten Fortsatz des Nachbarpins bilden. Mit anderen Worten umfasst jeweils einer der Pins zwei zweite Fortsätze, die den ersten Fortsatz eines benachbarten Pins in Pinlängsrichtung einschließen. Die zweiten Fortsätze des einen Pins bilden Längsanschläge für den ersten Fortsatz des anderen Pins, sodass dessen Bewegung in beide Pinlängsrichtung unterbunden ist. Bevorzugt greift der erste Fortsatz des einen Pins in die Ausnehmung des benachbarten Pins derart ein, dass der erste Fortsatz mit den zweiten Fortsätzen in Pinlängsrichtung, insbesondere spielfrei, in Kontakt steht.

Der erste Fortsatz und/oder die zweiten Fortsätze sind vorzugsweise ausgehend von einer Pinflanke konisch ausgebildet. Mit anderen Worten bilden die Fortsätze Zähne, die zu einer Zahnspitze hin verjüngt sind. Dies hat den Vorteil, dass sich die Pins beim Aneinanderpressen selbständig in die erwünschte axiale Lage ziehen und somit eine exakte Längsausrichtung der Pins zueinander erfolgt.

Es ist von Vorteil, wenn der erste Fortsatz und/oder die beiden zweiten Fortsätze jeweils ein freies Ende aufweisen, das abgerundet ist. Dies hat insbesondere den Vorteil, dass im Gebrauch das Risiko einer Verletzung des Blutgefäßes verringert ist. Durch die Abrundungen weisen die Fortsätze eine atraumatische Form auf.

Bei einer bevorzugten Ausführungsform weist die medizinische Vorrichtung wenigstens eine Hülle auf, die die Pins in dem zweiten Längsabschnitt, insbesondere im Bereich der Fortsätze einhüllt. Die Hülle kann durch wenigstens eine Kleberaupe gebildet sein. Alternativ oder zusätzlich kann die Hülle wenigstens eine geklebte Hülse und/oder wenigstens ein Coil, insbesondere eine Drahtwendel, umfassen. Die Hülle umschließt bevorzugt das absolute distale Ende der medizinischen Vorrichtung. Dies hat den Vorteil, dass im Gebrauch ein Verletzungsrisiko des Blutgefäßes durch die medizinische Vorrichtung weiter reduziert ist.

Es ist möglich, dass die Hülle neben dem zweiten Längsabschnitt auch den ersten Längsabschnitt der Pins einhüllt. Der erste und/oder zweite Längsabschnitt der Pins kann durch die Hülle teilweise oder vollständig eingehüllt sein. Bspw. kann die Hülle zusätzlich das Verbindungselement und/oder die wenigstens eine Schweißnaht umhüllen.

Vorzugsweise sind die Pins mit den Stegen der Gitterstruktur einstückig verbunden bzw. ausgebildet. Die Gitterstruktur ist bevorzugt aus einem Stück gebildet. Dies erfolgt vorzugsweise durch Laserscheiden der Gitterstruktur aus einem rohrförmigen Ausgangsmaterial, insbesondere Formgedächtnismaterial.

Nach einem nebengeordneten Aspekt betrifft die Erfindung ein Verfahren zur Herstellung einer medizinischen Vorrichtung, insbesondere eines Thrombektomie-Devices, die eine rohrförmige Gitterstruktur umfasst, die von einem radial komprimierten Zustand in einen radial expandierten Zustand überführbar ist und eine Vielzahl von zellenbildenden Stegen aufweist, wobei die Gitterstruktur an einem distalen Endabschnitt wenigstens zwei mit den Stegen verbundene Pins umfasst, wobei die Pins in einem ersten Längsabschnitt zur Fixierung der Pinquerschnittlage zumindest abschnittsweise flächig aneinander angepresst werden und die Pins in einem zweiten Längsabschnitt zur Fixierung der Pinlängslage miteinander formschlüssig verbunden werden, insbesondere ineinander eingreifen.

Bei einer bevorzugten Ausführungsform werden die Pins, insbesondere die gesamte Gitterstruktur, durch exzentrisches Laserschneiden, insbesondere unter Verwendung einer 4-Achs-Laserschneidvorrichtung, hergestellt. Dies hat den Vorteil, dass auf den Innenseiten der Pins schrägverlaufende Anpressflächen herstellbar sind, sodass ein optimales Positionieren der Pins aneinander möglich ist.

Zu den Vorteilen des Herstellungsverfahrens wird auf die im Zusammenhang mit der medizinischen Vorrichtung erläuterten Vorteile verwiesen. Darüber hinaus kann das Herstellungsverfahren alternativ oder zusätzlich einzelne oder eine Kombination mehrerer zuvor in Bezug auf die medizinische Vorrichtung genannte Merkmale aufweisen.

Die Erfindung wird nachstehend mit weiteren Einzelheiten unter Bezug auf die beigefügten Zeichnungen näher erläutert. Die dargestellten Ausführungsformen stellen Beispiele dar, wie die erfindungsgemäße Vorrichtung ausgestaltet sein kann.

In diesen zeigen,
- Fig. 1: eine teilweise Seitenansicht einer medizinischen Vorrichtung nach einem bevorzugten erfindungsgemäßen Ausführungsbeispiel;
- Fig. 2: eine vergrößerte Innenansicht eines Pins im Bereich eines distalen Endabschnitts einer Gitterstruktur der medizinischen Vorrichtung nach Fig. 1;
- Fig. 3: eine perspektivische Ansicht des distalen Endabschnitts der Gitterstruktur der medizinischen Vorrichtung nach Fig. 1 im gecrimpten Zustand;
- Fig. 4: einen Querschnitt durch einen ersten Längsabschnitt der Pins der medizinischen Vorrichtung nach Fig. 1 mit einer geschlossenen Crimphülse;
- Fig. 5: einen Querschnitt durch einen ersten Längsabschnitt der Pins einer weiteren erfindungsgemäßen medizinischen Vorrichtung mit einer offenen Crimphülse;
- Fig. 6: eine perspektivische Ansicht der medizinischen Vorrichtung nach Fig. 1 im Bereich des distalen Endabschnitts der Gitterstruktur; und
- Fig. 7: eine Seitenansicht der medizinischen Vorrichtung in einem radial komprimierten Zustand.

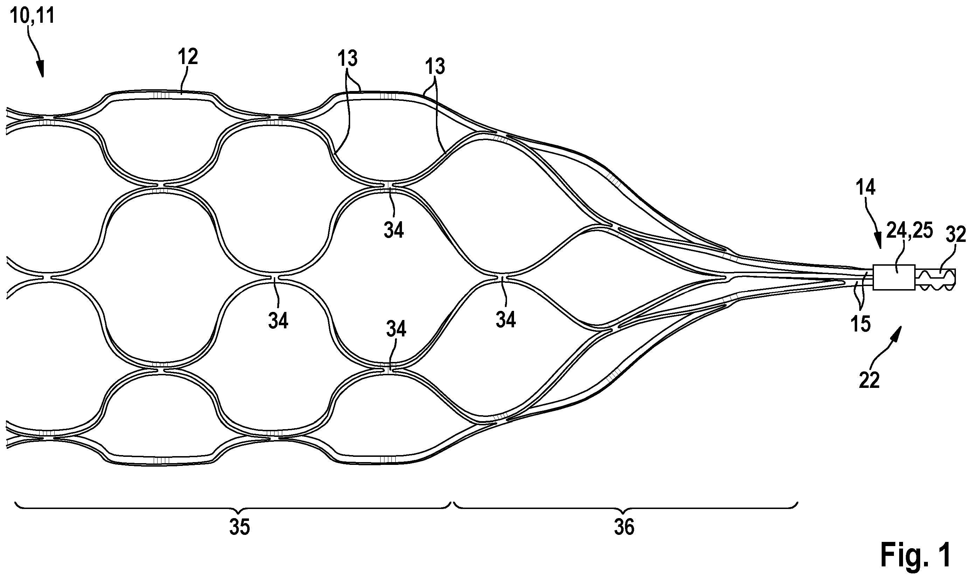

Fig. 1 zeigt eine medizinische Vorrichtung 10 nach einem bevorzugten erfindungsgemäßen Ausführungsbeispiel. Die medizinische Vorrichtung 10 ist ein Thrombektomie-Device 11 zur intravaskulären Behandlung. Das Thrombektomie-Device 11 dient zum Entfernen eines Thrombus aus einem Blutgefäß. Die medizinische Vorrichtung 10 kann auch als Stent-Retriever bezeichnet werden. In der folgenden Beschreibung wird die medizinische Vorrichtung 10 generell als Thrombektomie-Device 11 bezeichnet.

Das Thrombektomie-Device 11 weist eine Gitterstruktur 12 auf, die aus miteinander verbundenen Stegen 13 gebildet ist. Die Stege 13 sind an Stegverbindern 34 miteinander gekoppelt, wobei die Stegverbinder 34 jeweils vier Stege 13 miteinander verbinden. Hierbei sind die Stegverbinder 34 bevorzugt Y-Verbinder. Es ist möglich, dass Stegverbinder 34 vorgesehen sind, die jeweils drei Stege 13 miteinander verbinden. Insbesondere können Stegverbinder 34 vorgesehen sein, die jeweils (nur) zwei Stege 13 miteinander verbinden. Dies kann beispielsweise bei kurzen Endzellen der Fall sein. Vorzugsweise ist die Gitterstruktur 12 zumindest abschnittsweise rotationssymmetrisch geformt ausgebildet. Die Gitterstruktur 12 ist radial komprimierbar sowie selbständig radial expandierend.

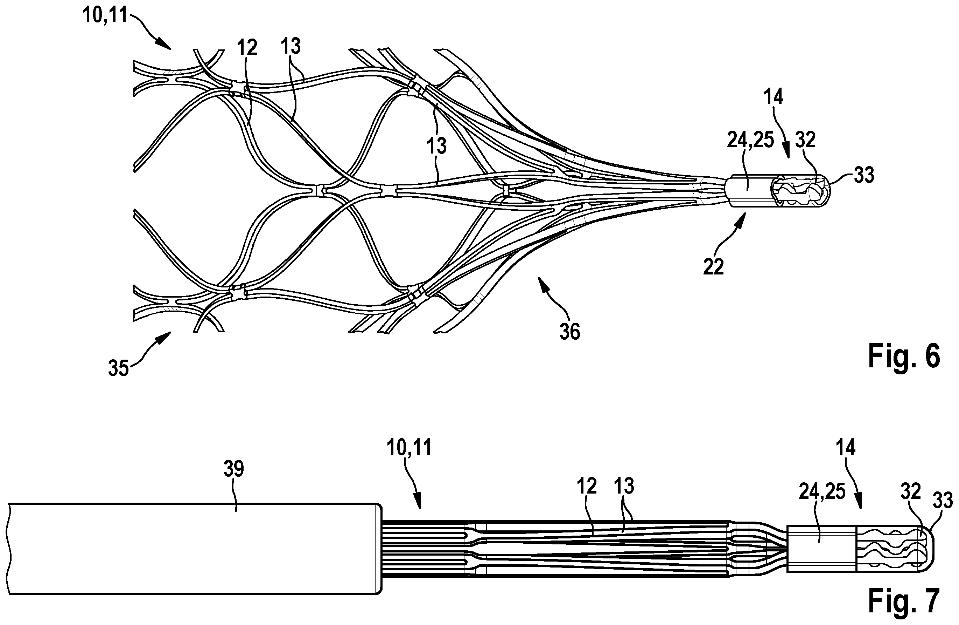

Wie in Fig. 1 und 6 erkennbar, ist die Gitterstruktur 12 teilweise rohrförmig ausgebildet. Mit anderen Worten weist die Gitterstruktur 12 einen rohrförmigen Bereich 35 auf. Die Gitterstruktur 12 weist eine Mittellängsachse auf. Zusätzlich umfasst die Gitterstruktur 12 einen konischen Bereich 36, der an den rohrförmigen Bereich 35 anschließt. Die Gitterstruktur 12 weist einen nicht dargestellten proximalen Endabschnitt und einen distalen Endabschnitt 14 auf. Unter dem distalen Endabschnitt 14 ist ein von einem Benutzer des Thrombektomie-Devices 11 abgewandtes Ende der Gitterstruktur 12 zu verstehen. Unter dem proximalen Endabschnitt ist ein dem Benutzer des Thrombektomie-Devices 11 zugewandtes Ende der Gitterstruktur 12 zu verstehen.

Der konische Bereich 36 läuft von den rohrförmigen Bereich 35 auf den distalen Endabschnitt 14 zu. Der distale Endabschnitt 14 bildet ein geschlossenes Ende der Gitterstruktur 12. Die Gitterstruktur 12 weist in dem distalen Endabschnitt 14 mehrere Pins 15 auf, die in distaler Richtung ein freies Ende haben. Auf die Pins 15 wird später näher eingegangen.

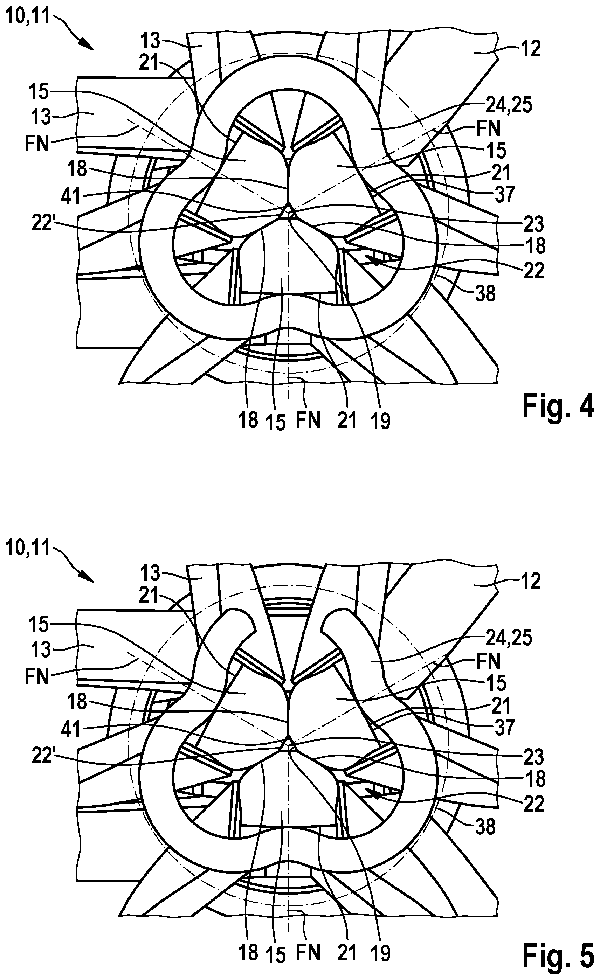

Die Gitterstruktur 12 ist aus einem Rohrmaterial lasergeschnitten. Das Rohrmaterial ist ein Formgedächtnismaterial. Konkret ist die Gitterstruktur 12 durch eine 4-Achs-Laserschneidanlage geschnitten, die einen exzentrischen Laserschnitt ermöglicht. Mit anderen Worten ist die Gitterstruktur 12 zumindest abschnittsweise durch einen exzentrischen Laserschnitt geschnitten. Dies bedeutet, dass der Laserstrahl beim Laserschneiden nicht die Mittellängsachse des Rohrmaterials schneidet. Dadurch können die Stege 13 und/oder die Pins 15 einen von einem standardmäßig trapezförmigen Querschnitt abweichenden Querschnitt aufweisen. Dies ist ein den Fig. 4 und 5 gut zu erkennen. Andere Geometrien der Gitterstruktur 12 sind möglich. Neben dem Laserschneiden sind andere Herstellungsverfahren möglich.

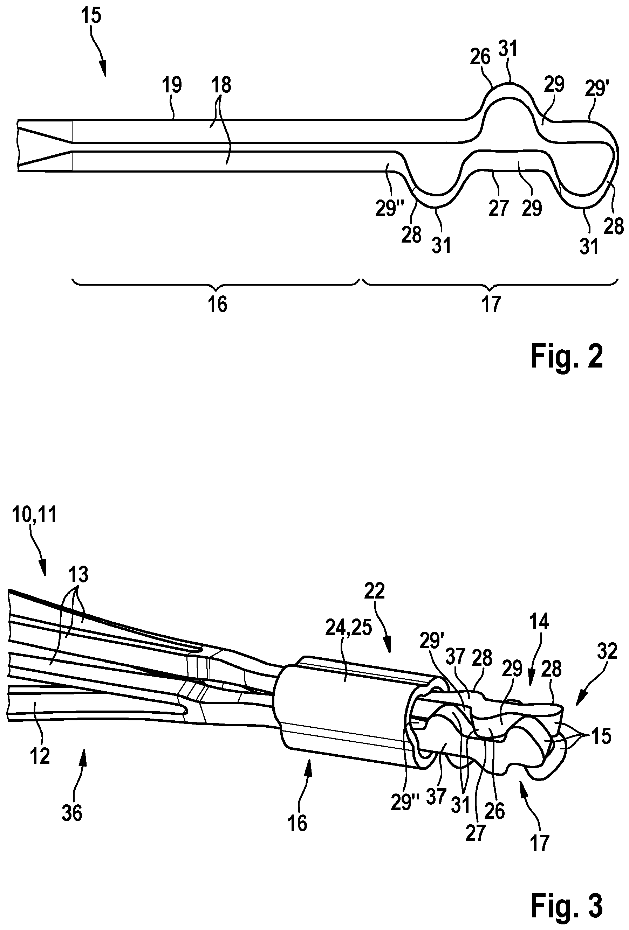

Wie vorstehend beschrieben, weist die Gitterstruktur 12 in dem distalen Endabschnitt 14 mehrere Pins 15 mit einem freien Ende auf. Gemäß Fig. 3, 4 und 5 ist gut zu erkennen, dass die Gitterstruktur 12 insgesamt drei Pins 15 aufweist. Alternativ kann die Gitterstruktur 12 auch zwei oder mehr als drei Pins 15 aufweisen.

Die Pins 15 bilden ein freies, distales Ende 32 der Gitterstruktur 12. Das freie Ende 32 der Gitterstruktur 12 ist radial, d.h. quer zur Mittellängsachse geschlossen. Mit anderen Worten weist die Gitterstruktur 12 ein geschlossenes distales Ende 32 auf. Dies ermöglicht im Gebrauch ein besseres Einfangen von Thrombusfragmenten und verhindert ein Wiederhinausgleiten eines bereits im Inneren der Gitterstruktur 12 aufgenommenen Thrombus.

Die Pins 15 bilden im distalen Endabschnitt 14 ein Bündel 22. Mit anderen Worten sind die Pins 15 in dem distalen Endabschnitt 14 zur Bildung des geschlossenen Endes radial gebündelt. Die Pins 15 erstrecken sich in dem distalen Endabschnitt 14 zumindest teilweise geradlinig. Die Pins 15 verlaufen parallel. Die Pins 15 erstrecken sich entlang der Mittellängsachse der Gitterstruktur 12. Oder anders gesagt, erstrecken sich die Pins 15 in einer Längsrichtung der Gitterstruktur 12.

Die Pins 15 sind Teil der Gitterstruktur 12. Konkret sind die Pins 15 mit den Stegen 13 der Gitterstruktur 12 einstückig, insbesondere monolithisch ausgebildet. Wie in Fig. 1, 3, 6 und 7 gezeigt ist, schließen die Pins 15 mit den Stegen 13 des konischen Bereichs 36 der Gitterstruktur 12 an.

Die Pins 15 weisen einen ersten Längsabschnitt 16 und einen zweiten Längsabschnitt 17 auf, der an den ersten Längsabschnitt 16 angrenzt. In dem ersten Längsabschnitt 16 sind die Pins 15 zur Fixierung der Pinquerschnittlage radial innen aneinander flächig angepresst. In dem zweiten Längsabschnitt 17 sind die Pins 15 zur Fixierung der Pinlängslage miteinander formschlüssig verbunden.

Um die Pins 15 aneinander zu pressen bzw. zu fixieren, weist das Thrombektomie-Device 11 ein Verbindungselement 24 auf. Das Verbindungselement 24 ist bevorzugt röntgensichtbar. Das Verbindungselement 24 weist einen im Wesentlichen runden Außenumfang 38 auf. Gemäß Fig. 4 und 5 ist das Verbindungselement 24 ein Crimphülse 25. Die Crimphülse 25 kann dabei in Umfangsrichtung geschlossen sein (Fig. 4) oder in Umfangsrichtung teilweise offen sein (Fig. 5). Mit anderen Worten kann die Crimphülse 25 gemäß Fig. 5 einen Längsschlitz aufweisen. Oder anders gesagt, ist die Crimphülse 25 längsgeteilt ausgebildet.

Die Crimphülse 25 ist in dem ersten Längsabschnitt 16 an den Pins 15 außen angeordnet. Die Crimphülse 25 ist mit den Pins 15 zum radialen Fixieren vercrimpt. Die Crimphülse 25 dient neben der Fixierung der Pins als Röntgenmarker.

Alternativ oder zusätzlich ist es möglich, dass das Verbindungselement 24 eine Klebehülse umfasst, die an den Pins 15 außen angeordnet ist und die Pins 15 in ihrer Querschnittslage fixiert. Hier sind die Pins 15 zum Fixieren durch die Klebehülse miteinander verklebt. Alternativ oder zusätzlich können die Pins 15 miteinander verschweißt sein. Beispielsweise können die Crimphülsen 25 gemäß Fig. 4 und 5 zusätzlich mit wenigstens einem der Pins 15 verschweißt sein. Es ist möglich, dass die Pins 15 durch wenigstens eine Schweißnaht miteinander verbunden sind. Des Weiteren ist es möglich, dass jeweils zwei benachbarte Pins 15 durch jeweils eine Schweißung, insbesondere durch wenigstens eine Schweißnaht oder wenigstens einen Schweißpunkt, miteinander verbunden sind.

Wie vorstehend beschrieben, sind die Pins 15 in dem ersten Längsabschnitt 16 flächig aneinander angepresst. Dazu weisen die Pins 15 jeweils auf einer radial innenliegenden Pinseite 19, insbesondere Innenseite, zwei Anpressflächen 18 auf. Die Anpressflächen 18 verlaufen entlang der Pinlängsrichtung in dem ersten Längsabschnitt 16. Es ist möglich, dass die beiden Anpressflächen 18 auch in dem zweiten Längsabschnitt 17 der Pins 15 verlaufen. Die Anpressflächen 18 sind auf der radial innenliegenden Pinseite 19 aufeinander zulaufend ausgebildet. Die Anpressflächen 18 dienen jeweils zur Anlage eines benachbarten Pins 15.

Wie in den Fig. 4 und 5 zu sehen ist, liegen jeweils zwei benachbarte Pins 15 mit jeweils einer Anpressfläche 18 aneinander an. Mit anderen Worten stehen jeweils zwei benachbarte Pins 15 durch die Anpressflächen 18 in Flächenkontakt. Die Anpressflächen 18 sind geradflächig ausgebildet. Die beiden Anpressflächen 18 des jeweiligen Pins 15 verlaufen in Pinlängsrichtung parallel. Die Anpressflächen 18 der Pins 15 sind so ausgerichtet, dass bei Einleitung einer radialer Anpresskraft der Crimphülse 25 die Pins 15 in ihrer Querschnittslage fixiert sind.

Des Weiteren weisen die Pins 15 jeweils eine radial außenliegende Pinseite 37, insbesondere Außenseite, auf, an der die Crimphülse 25 anliegt. Um die Anpresskraft bzw. Haltekraft der Crimphülse 25 in den jeweiligen Pin 15 einzuleiten, weisen die Pins 15 jeweils eine radial außenliegende Krafteinleitfläche 21, die in dem ersten Längsabschnitt 16 in Pinlängsrichtung verläuft. Mit anderen Worten ist auf der radial außenliegenden Pinseite 37 die Krafteinleitfläche 21 ausgebildet. Die Krafteinleitfläche 21 ist den beiden radial innenliegende Anpressflächen 18 gegenüber angeordnet. Die beiden Anpressflächen 18 verlaufen in Bezug auf die Krafteinleitfläche 21 schräg.

Die radial außenliegende Pinseite 37 der Pins 15 ist die jeweils voneinander abgewandte Seite der Pins 15 quer zur Pinlängsrichtung, insbesondere quer zu Mittellängsachse. Über die Krafteinleitfläche 21 wird im fixierten Zustand der Pins 15 eine Anpresskraft der Crimphülse 25 von außen in die Pins 15 eingeleitet, sodass ein Aneinanderpressen der Anpressflächen 18 zweier benachbarter Pins 15 erfolgt. Durch die schrägverlaufende Anpressflächen 18 wird eine Teilkomponente, insbesondere gleich große Teilkomponenten, der eingeleiteten Anpresskraft in Richtung der Anpressflächen 18 umgelenkt, sodass gleichmäßige Verteilung der Anpresskraft auf die beiden Anpressflächen 18 erfolgt.

Die Krafteinleitfläche 21 verläuft über die gesamte Länge des ersten Längsabschnitts 16 der Pins 15. Dies kann alternativ abschnittsweise erfolgen. Die Krafteinleitfläche 21 weist eine Krümmung auf. Mit anderen Worten ist die Krafteinleitfläche 21 gewölbt ausgebildet. Wie in Fig. 4 und 5 erkennbar, ist die Wölbung bzw. Krümmung konvex.

Ebenfalls zeigen die Fig. 4 und 5, dass die Pins 15 sternförmig angeordnet sind. Des Weiteren ist zu sehen, dass die Pins 15 im Querschnitt ringsegmentförmig ausgebildet sind. Mit anderen Worten umfassen die Pins 15 im Querschnitt jeweils ein Ringsegment. Die Ringsegmente decken in Umfangsrichtung jeweils einen Winkelbereich von zirka 120 Grad ab.

Die Pins 15 weisen dabei ein gemeinsames Zentrum 22' auf, das radial innenliegt. Durch das gemeinsame Zentrum 22' verläuft die Mittellängsachse der Gitterstruktur 12. Das gemeinsame Zentrum 22' ist durch einen Mittelbereich gebildet, der zwischen den radial innenliegenden Pinseiten 19 vorgesehen ist. Gemäß Fig. 4 und 5 ist das gemeinsame Zentrum 22' ein Freiraum, der zwischen den radial innenliegenden Pinseiten 19 der Pins 15 gebildet ist. Die Pins 15 weisen an deren radial innenliegenden Pinseiten 19 eine abgeflachte Spitze 41 auf. Mit anderen Worten weisen die Pins 15 auf deren radial innenliegenden Pinseiten 19 jeweils eine Schmalfläche auf. Die beiden radial innenliegenden Anpressflächen 18 laufen auf die Schmalfläche zu. Die beiden Anpressflächen 18 schließen radial innen an die Schmalfläche an. Die Schmalfläche verläuft in Pinlängsrichtung zumindest im ersten Pinlängsabschnitt 16. Die Schmalfläche ist ein Abschnitt eines Innendurchmessers eines geschnittenen, insbesondere lasergeschnittenen, rohrförmigen Ausgangsmaterials. Aufgrund der radial innen abgeflachten Spitzen 41 der Pins 15 ist das gemeinsame Zentrum 22' im Querschnitt im Wesentlichen dreieckförmig.

Die Pins 15 sind derart miteinander angeordnet, dass eine Flächennormale FN der Krafteinleitflächen 21 des jeweiligen Pins 15 durch das gemeinsame Zentrum 22' verläuft. Die Flächennormale FN ist eine gedachte Gerade, die normal zu der Krafteinleitfläche 21 verläuft. Die Flächennormale FN entspricht der Wirklinie der eingeleiteten Anpresskraft im Bereich der Krafteinleitfläche 21. Im konkreten Fall, der den Idealfall darstellt, schneiden sich die Flächennormalen FN der Krafteinleitflächen 21 der Pins 15 in einem gemeinsamen Mittelpunkt 23, der auf der Mittellängsachse der Gitterstruktur 12 liegt. Es ist möglich, dass die Flächennormalen FN der Krafteinleitflächen 21 der Pins 15 annähernd, d.h. in geringem Abstand, um den gemeinsamen Mittelpunkt 23 verlaufen. Dies stellt den Realfall dar.

Wie vorstehend beschrieben, sind die Pins 15 in dem zweiten Längsabschnitt 17 miteinander formschlüssig verbunden. Gemäß Fig. 2 und 3 ist gezeigt, dass die Pins 15 in dem zweiten Längsabschnitt 17 jeweils einen ersten Fortsatz 26 und eine gegenüber angeordnete Ausnehmung 27 aufweisen. Der erste Fortsatz 26 ist an einer ersten Pinflanke 29' des jeweiligen Pins 15 angeordnet. Die Ausnehmung 27 ist an einer zweiten Pinflanke 29" ausgebildet, die der ersten Pinflanke 29' gegenüberliegt. Der erste Fortsatz 26 des jeweiligen Pins 15 greift in die Ausnehmung 27 des jeweils benachbarten Pins 15 ein. Mit anderen Worten ragt der erste Fortsatz 26 des jeweiligen Pins 15 in die Ausnehmung 27 des jeweils benachbarten Pins 15 ein. Die Ausnehmung 27 bildet eine Aufnahme für den ersten Fortsatz 26 eines der benachbarten Pins 15. Die Ausnehmung 27 ist in Pinlängsrichtung von zwei zweiten Fortsätzen 28 begrenzt. Die zweiten Fortsätze 28 bilden jeweils einen Längsanschlag für den eingreifenden ersten Fortsatz 26 des benachbarten Pins 15. Dadurch ist die Längsposition der Pins 15 zueinander festgelegt.

Der erste Fortsatz 26 und die zweiten Fortsätze 28 der Pins 15 sind ausgehend von der jeweiligen Pinflanke 29', 29" konisch ausgebildet. Die Fortsätze 26, 28 sind zahnartig ausgebildet. Der erste Fortsatz 26 und die zweiten Fortsätze 28 bilden eine Verzahnung zur Fixierung der Längslage der Pins 15. Der erste Fortsatz 26 und die zweiten Fortsätze 28 erstrecken sich in entgegengesetzter Richtung an dem jeweiligen Pin 15. Die Fortsätze 26, 28 erstrecken sich in einer Umfangsrichtung des Bündels 22 der Pins 15. Die Fortsätze 26, 28 der Pins 15 weisen jeweils ein freies Ende 31 auf, das abgerundet ist. Mit anderen Worten weisen die Fortsätze 26, 28 runde Spitzen somit eine atraumatische Form auf.

Wie in den Fig. 6 und 7 gezeigt, weist das Thrombektomie-Device 11 eine Hülle 33 auf, die die Pins 15 in dem zweiten Längsabschnitt 17 umhüllt. Konkret hüllt die Hülle 33 die Fortsätze 26, 28 und die Ausnehmungen 27 ein. Die Hülle 33 kann aus einer Kleberaupe gebildet sein. Alternativ kann die Hülle 33 auf das freie Ende der Pins 15 aufgeschoben und mit diesen verklebt sein. Die Hülle 33 und die Crimphülse 25 weisen im Wesentlichen einen gleichen Außenumfang 38 auf.

Des Weiteren zeigt Fig. 7 das Thrombektomie-Device 11 beim Einschieben bzw. Herausschieben aus einem Katheter 39. Die Gitterstruktur 12 des Thrombektomie-Devices 11 befindet sich in einem radial komprimierten Zustand. Aus Fig. 7 ist gut zu erkennen, dass durch die exakte radiale und axiale Lage der Pins 15 zueinander ein problemloses Bewegen des komprimierten Thrombektomie-Devices 11 in den Katheter sowie aus dem Katheter ermöglicht ist.

### Bezugszeichenliste

- 10: Medizinische Vorrichtung
- 11: Thrombektomie-Device
- 12: rohrförmige Gitterstruktur
- 13: zellenbildende Stege
- 14: distaler Endabschnitt der Gitterstruktur
- 15: Pins
- 16: erster Längsabschnitt der Pins
- 17: zweiter Längsabschnitt der Pins
- 18: Anpressfläche
- 19: radial innenliegende Pinseite
- 21: Krafteinleitfläche
- 22: Bündel
- 22': gemeinsames Zentrum
- 23: gemeinsamer Mittelpunkt
- 24: Verbindungselement
- 25: Crimphülse
- 26: erster Fortsatz
- 27: Ausnehmung
- 28: zweite Fortsätze
- 29': erste Pinflanke
- 29": zweite Pinflanke
- 31: freies Ende der Fortsätze
- 32: geschlossenes Ende
- 33: Hülle
- 34: Stegverbinder
- 35: rohrförmiger Bereich
- 36: konischer Bereich
- 37: radial außenliegende Pinseite
- 38: Außenumfang
- 39: Katheter
- 41: abgeflachte Spitze
- FN: Flächennormale

## Patentansprüche

1. Medizinische Vorrichtung (10) zur intravaskulären Behandlung, insbesondere Thrombektomie-Device (11), mit einer zumindest abschnittsweise rohrförmigen Gitterstruktur (12), die von einem radial komprimierten Zustand in einen radial expandierten Zustand überführbar ist und eine Vielzahl von zellenbildenden Stegen (13) aufweist, wobei die Gitterstruktur (12) an einem distalen Endabschnitt (14) wenigstens zwei, insbesondere drei oder mehr, mit den Stegen (13) verbundene Pins (15) umfasst, die ein freies Ende aufweisen,
**dadurch gekennzeichnet, dass**
die Pins (15) in einem ersten Längsabschnitt (16) zur Fixierung der Pinquerschnittslage zumindest abschnittsweise flächig aneinander angepresst sind und/oder die Pins (15) in einem zweiten Längsabschnitt (17) zur Fixierung der Pinlängslage miteinander formschlüssig verbunden sind, insbesondere ineinander eingreifen.

2. Vorrichtung (10) nach Anspruch 1,
**dadurch gekennzeichnet, dass**
die Pins (15) jeweils wenigstens eine radial innenliegende Anpressfläche (18) aufweisen, mit der die Pins (15) aneinander anliegen, wobei die Anpressflächen (18) so ausgerichtet sind, dass bei Einleitung einer Anpresskraft die Pins (15) in ihrer Querschnittslage fixiert sind.

3. Vorrichtung (10) nach Anspruch 1 oder 2,
**dadurch gekennzeichnet, dass**
die Pins (15) jeweils auf einer radial innenliegenden Pinseite (19) zwei aufeinander zulaufende Anpressflächen (18) zur Anlage zumindest eines benachbarten Pins (15) aufweisen.

4. Vorrichtung (10) nach einem der vorhergehenden Ansprüche, insbesondere Anspruch 2 oder 3,
**dadurch gekennzeichnet, dass**
die Pins (15) wenigstens eine radial außen liegende Krafteinleitfläche (21) aufweisen, wobei eine/die radial innenliegende Anpressfläche (18), insbesondere die beiden Anpressflächen, in Bezug auf die Krafteinleitfläche (21) schräg verläuft.

5. Vorrichtung (10) nach einem der vorhergehenden Ansprüche, insbesondere nach Anspruch 4,
**dadurch gekennzeichnet, dass**
die Pins (15) ein gemeinsames Zentrum (22'), insbesondere einen gemeinsamen Mittelpunkt (23), umfassen, das radial innenliegt, wobei zumindest eine Flächennormale FN der Krafteinleitfläche (21) des jeweiligen Pins (15) durch das gemeinsame Zentrum (22') verläuft.

6. Vorrichtung (10) nach einem der vorhergehenden Ansprüche,
**dadurch gekennzeichnet, dass**
die Pins wenigstens eine gemeinsame Außenkontur aufweisen, die im Querschnitt annähernd einem regelmäßigen Polygon entspricht und/oder die Pins (15) miteinander sternförmig angeordnet sind.

7. Vorrichtung (10) nach einem der vorhergehenden Ansprüche,
**dadurch gekennzeichnet, dass**
die Pins (15) im Querschnitt ring- oder kreissegmentförmig ausgebildet sind.

8. Vorrichtung (10) nach einem der vorhergehenden Ansprüche,
**gekennzeichnet durch**,
wenigstens ein Verbindungselement (24), insbesondere eine Crimphülse (25) und/oder eine geklebte Hülse, das an den Pins (15) außen angeordnet ist und die Pins (15), insbesondere in dem ersten Längsabschnitt (16), radial aneinander anpresst.

9. Vorrichtung (10) nach einem der vorhergehenden Ansprüche,
**dadurch gekennzeichnet, dass**
das Verbindungselement (24) einen Außenumfang (38) aufweist, der im Wesentlichen kreisförmig, insbesondere rund ist und/oder das Verbindungselement (24) umlaufend geschlossen oder teilweise offen ist.

10. Vorrichtung (10) nach einem der vorhergehenden Ansprüche,
**gekennzeichnet durch**,
die Pins **durch** wenigstens eine Schweißnaht, insbesondere in dem ersten Längsabschnitt (16), miteinander stoffschlüssig verbunden sind.

11. Vorrichtung (10) nach einem der vorhergehenden Ansprüche,
**dadurch gekennzeichnet, dass**
die Pins (15), insbesondere in dem zweiten Längsabschnitt (17), jeweils wenigstens einen ersten Fortsatz (26) und wenigstens eine gegenüber angeordnete Ausnehmung (27) aufweisen, wobei der erste Fortsatz (26) des einen Pins (15) in die Ausnehmung (27) des benachbarten Pins (15) zumindest abschnittsweise eingreift, und/oder die Ausnehmung (27) in Längsrichtung des Pins (15) von wenigstens zwei zweiten Fortsätzen (28) begrenzt ist, die jeweils einen Längsanschlag für den ersten Fortsatz (26) bilden.

12. Vorrichtung (10) nach Anspruch 11,
**dadurch gekennzeichnet, dass**
der erste Fortsatz (26) und/oder die zweiten Fortsätze (28) ausgehend von einer Pinflanke (29', 29") konisch ausgebildet sind, und/oder der erste Fortsatz (26) und/oder die beiden zweiten Fortsätze (28) jeweils ein freies Ende (31) aufweisen, das abgerundet ist.

13. Vorrichtung (10) nach einem der vorhergehenden Ansprüche,
**dadurch gekennzeichnet, dass**
wenigstens eine Hülle (33) vorgesehen ist, die die Pins (15) in dem zweiten Längsabschnitt (17), insbesondere im Bereich der Fortsätze (26, 28) einhüllt.

14. Vorrichtung (10) nach einem der vorhergehenden Ansprüche,
**dadurch gekennzeichnet, dass**
die Pins (15), insbesondere ein Bündel (22) der Pins (15), ein geschlossenes Ende (32) der Gitterstruktur (12) bilden und/oder die Pins (15) mit den Stegen (13) der Gitterstruktur (12) einstückig verbunden sind.

15. Verfahren zur Herstellung einer medizinischen Vorrichtung (10), insbesondere eines Thrombektomie-Devices (11), die eine rohrförmige Gitterstruktur (12) umfasst, die von einem radial komprimierten Zustand in einen radial expandierten Zustand überführbar ist und eine Vielzahl von zellenbildenden Stegen (13) aufweist, wobei die Gitterstruktur (12) an einem distalen Endabschnitt (14) wenigstens zwei mit den Stegen (13) verbundene Pins (15) umfasst, wobei die Pins (15) in einem ersten Längsabschnitt (16) zur Fixierung der Pinquerschnittslage zumindest abschnittsweise flächig aneinander angepresst werden und die Pins (15) in einem zweiten Längsabschnitt (17) zur Fixierung der Pinlängslage miteinander formschlüssig verbunden werden, insbesondere ineinander eingreifen.

## Claims

1. A medical device (10) for intravascular treatment, in particular a thrombectomy device (11), with a mesh structure (12) which is tubular at least in sections and which can be transitioned from a radially compressed state into a radially expanded state and which has a plurality of cell-forming webs (13), wherein at a distal end section (14), the mesh structure (12) comprises at least two, in particular three or more, pins (15) which have a free end and which are connected to the webs (13),
**characterized in that**
in a first longitudinal section (16), the pins (15) are pressed flat against each other at least in sections in order to fix the cross-sectional position of the pin, and/or in a second longitudinal section (17), the pins (15) are positively locked with each other, in particular interengaging, in order to fix the longitudinal position of the pin.

2. The device (10) as claimed in claim 1,
**characterized in that**
the pins (15) respectively have at least one radially internally disposed contact surface (18) with which the pins (15) bear against each other, wherein the contact surfaces (18) are orientated in a manner such that when a contact force is introduced, the pins (15) are fixed in their cross-sectional position.

3. The device (10) as claimed in claim 1 or claim 2,
**characterized in that**
on a radially internally disposed pin side (19), the pins (15) respectively have two converging contact surfaces (18) for making contact with at least one adjacent pin (15).

4. The device (10) as claimed in one of the preceding claims, in particular claim 2 or claim 3,
**characterized in that**
the pins (15) have at least one radially externally disposed force introduction surface (21), wherein a/the radially inner contact surface (18), in particular the two contact surfaces, extends obliquely with respect to the force introduction surface (21).

5. The device (10) as claimed in one of the preceding claims, in particular as claimed in claim 4,
**characterized in that**
the pins (15) comprise a common centre (22'), in particular a common centre point (23), which is radially internally disposed, wherein at least one normal FN to the surface of the force introduction surface (21) of the respective pin (15) passes through the common centre (22').

6. The device (10) as claimed in one of the preceding claims,
**characterized in that**
the pins (15) have at least one common external contour which approximately corresponds to a regular polygon in cross-section, and/or the pins (15) are disposed in a star shape with respect to each other.

7. The device (10) as claimed in one of the preceding claims,
**characterized in that**
in cross-section, the pins (15) are ring-shaped or circular segment-shaped.

8. The device (10) as claimed in one of the preceding claims,
**characterized by**
at least one connecting element (24), in particular a crimp sleeve (25) and/or an adhesive sleeve, which is disposed on the exterior of the pins (15) and presses the pins (15) radially against each other, in particular in the first longitudinal section (16).

9. The device (10) as claimed in one of the preceding claims,
**characterized in that**
the connecting element (24) has an external perimeter (38) which is substantially circular, in particular round, and/or the connecting element (24) is completely closed or partially open.

10. The device (10) as claimed in one of the preceding claims,
**characterized by**
the pins (15) being connected together in a material-bonded manner by at least one weld seam, in particular in the first longitudinal section (16).

11. The device (10) as claimed in one of the preceding claims,
**characterized in that**
in particular in the second longitudinal section (17), the pins (15) respectively have at least one first projection (26) and at least one oppositely disposed recess (27), wherein the first projection (26) of one pin (15) engages at least in sections in the recess (27) of the adjacent pin (15), and/or in the longitudinal direction of the pin (15), the recess (27) is delimited by at least two second projections (28), which respectively form a longitudinal stop for the first projection (26).

12. The device (10) as claimed in claim 11,
**characterized in that**
starting from a pin flank (29', 29"), the first projection (26) and/or the second projections (28) are tapered in construction, and/or the first projection (26) and/or the two second projections (28) respectively have a free end (31) which is rounded.

13. The device (10) as claimed in one of the preceding claims,
**characterized in that**
at least one cover (33) is provided, which envelops the pins (15) in the second longitudinal section (17), in particular in the region of the projections (26, 28).

14. The device (10) as claimed in one of the preceding claims,
**characterized in that**
the pins (15), in particular a bundle (22) of the pins (15), form a closed end (32) of the mesh structure (12), and/or the pins (15) are connected into one piece with the webs (13) of the mesh structure (12).

15. A method for the production of a medical device (10), in particular a thrombectomy device (11), which comprises a tubular mesh structure (12) which can be transitioned from a radially compressed state into a radially expanded state and which has a plurality of cell-forming webs (13), wherein at a distal end section (14), the mesh structure (12) comprises at least two pins (15) connected to the webs (13), wherein in a first longitudinal section (16), the pins (15) are pressed flat against each other at least in sections in order to fix the cross-sectional position of the pin, and in a second longitudinal section (17), the pins (15) are positively locked with each other, in particular interengaging, in order to fix the longitudinal position of the pin.

## Revendications

1. Dispositif médical (10) pour le traitement intravasculaire, en particulier dispositif de thrombectomie (11), avec une structure en treillis (12) au moins partiellement tubulaire, qui peut être passée d'un état radialement comprimé à un état radialement dilaté et présente une diversité de traverses (13) formant des cellules, la structure en treillis (12) sur une section terminale distale (14) comprenant au moins deux broches (15), en particulier trois ou plus, reliées aux traverses (13) et qui présentent une extrémité libre,
**caractérisé en ce que**
les broches (15), dans une première section longitudinale (16), pour fixer la position transversale des broches, sont, au moins par sections, serrées les unes contre les autres et/ou les broches (15), dans une seconde section longitudinale (17), sont jointes par correspondance géométrique pour fixer la position longitudinale des broches, en particulier sont en prise les unes dans les autres.

2. Dispositif (10) selon la revendication 1,
**caractérisé en ce que**
les broches (15) présentent chacune au moins une surface de compression interne radiale (18) par laquelle les broches (15) sont adjacents les unes aux autres, les surfaces de compression (18) étant orientées de telle sorte que, lors de l'introduction d'une force de serrage, les broches (15) soient fixées dans leur position de section transversale.

3. Dispositif (10) selon la revendication 1 ou 2,
**caractérisé en ce que**
les broches (15) présentent chacune, sur un côté radialement interne de la broche (19), deux surfaces de compression (18) convergeant l'une vers l'autre destinées à être en contact avec au moins une broche voisine (15).

4. Dispositif (10) selon l'une des revendications précédentes, notamment la revendication 2 ou 3,
**caractérisé en ce que**
les broches (15) présentent au moins une surface d'entrée de force radialement extérieure (21), une/la surface de contact radialement intérieure (18), en particulier les deux surfaces de compression, s'étendant à l'oblique par rapport à la surface d'entrée de force (21).

5. Dispositif (10) selon l'une des revendications précédentes, notamment selon la revendication 4,
**caractérisé en ce que**
les broches (15) comprennent un centre commun (22'), et notamment un point central commun (23), situé radialement à l'intérieur, avec au moins une surface normale FN de la surface d'entrée de force (21) de la broche respective (15) passant par le centre commun (22').

6. Dispositif (10) selon l'une des revendications précédentes,
**caractérisé en ce que**
les broches (15) présentent au moins un contour extérieur commun qui correspond approximativement à un polygone régulier en section transversale et/ou les broches (15) sont disposées ensemble en forme d'étoile.

7. Dispositif (10) selon l'une des revendications précédentes,
**caractérisé en ce que**
les broches (15) ont une conformation en anneau ou en arc de cercle dans la section transversale.

8. Dispositif (10) selon l'une des revendications précédentes,
**caractérisé par**
au moins un élément de liaison (24), en particulier un manchon serti (25) et/ou une enveloppe collée, qui est placé(e) à l'extérieur des broches (15) et comprime radialement les broches (15) radialement les unes contre les autres, en particulier dans la première section longitudinale (16).

9. Dispositif (10) selon l'une des revendications précédentes,
**caractérisé en ce que**
l'élément de liaison (24) présente une circonférence extérieure (38) qui est sensiblement circulaire, en particulier ronde et/ou que l'élément de liaison (24) est fermé ou partiellement ouvert sur sa circonférence.

10. Dispositif (10) selon l'une des revendications précédentes,
**caractérisé en ce que**
les broches (15) sont reliées entre elles par correspondance de matière par au moins un cordon de soudure, en particulier dans la première section longitudinale (16).

11. Dispositif (10) selon l'une des revendications précédentes,
**caractérisé en ce que**
les broches (15), en particulier dans la seconde section longitudinale (17), comportent respectivement au moins un premier prolongement (26) et au moins un évidement disposé à l'opposé (27), le premier prolongement (26) de l'une des broches (15) étant au moins partiellement en prise dans l'évidement (27) de la broche voisine (15), et/ou l'évidement (27) étant limité dans le sens longitudinal de la broche (15) par au moins deux seconds prolongements (28), chacun constituant une butée longitudinale pour le premier prolongement (26).

12. Dispositif (10) selon la revendication 11,
**caractérisé en ce que**
le premier prolongement (26) et/ou les seconds prolongements (28) ont une conformation conique à partir d'un flanc de broche (29', 29") et/ou que le premier prolongement (26) et/ou les deux seconds prolongements (28) présentent chacun une extrémité libre (31) qui est arrondie.

13. Dispositif (10) selon l'une des revendications précédentes,
**caractérisé en ce que**
qu'au moins une enveloppe (33) est prévue pour envelopper les broches (15) dans la seconde section longitudinale (17), en particulier au niveau des prolongements (26, 28).

14. Dispositif (10) selon l'une des revendications précédentes,
**caractérisé en ce que**
les broches (15), en particulier un faisceau (22) de broches (15), forment une extrémité fermée (32) de la structure en treillis (12) et/ou que les broches (15) sont reliées en formant une seule pièce aux traverses (13) de la structure en treillis (12).

15. Procédé de fabrication d'un dispositif médical (10), en particulier d'un dispositif de thrombectomie (11), comprenant une structure tubulaire en treillis (12), qui peut être passée d'un état radialement comprimé à un état radialement dilaté et présente une diversité de traverses (13) formant des cellules, la structure en treillis (12) comprenant, à une extrémité terminale distale (14), au moins deux broches (15) reliées aux traverses (13), les broches (15) étant comprimées au moins partiellement les unes contre les autres dans une première section longitudinale (16) pour fixer la section transversale des broches et les broches (15), dans une seconde section longitudinale (17), pour fixer la position longitudinale des broches, étant jointes les unes aux autres en correspondance géométrique, en particulier étant en prise les unes dans les autres.
